(19) **Europäisches Patentamt / European Patent Office / Office européen des brevets**

(11) **EP 3 175 918 A1**

(12) **EUROPEAN PATENT APPLICATION**
published in accordance with Art. 153(4) EPC

(43) Date of publication:
**07.06.2017 Bulletin 2017/23**

(21) Application number: **15827560.2**

(22) Date of filing: **23.07.2015**

(51) Int Cl.:
**B01J 23/62** (2006.01)    **B01J 23/63** (2006.01)
**C07C 4/06** (2006.01)    **B01J 37/02** (2006.01)

(86) International application number:
**PCT/KR2015/007671**

(87) International publication number:
**WO 2016/017994 (04.02.2016 Gazette 2016/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **28.07.2014 KR 20140095906**

(71) Applicant: **Lotte Chemical Corporation of (Sindaebang-dong)**
**Dongjak-gu**
**Seoul 156-711 (KR)**

(72) Inventors:
• **LEE, Jaeyeon**
  **Daejeon 305-720 (KR)**
• **KIM, Inae**
  **Daejeon 305-720 (KR)**
• **KANG, Seunghee**
  **Daejeon 305-720 (KR)**
• **SEO, Youngjong**
  **Daejeon 305-707 (KR)**

(74) Representative: **Swindell & Pearson Limited**
**48 Friar Gate**
**Derby DE1 1GY (GB)**

(54) **DEHYDROGENATION CATALYST, AND PREPARATION METHOD THEREFOR**

(57) Disclosed is a dehydrogenation catalyst for converting the C4 LPG, isobutane, and the C3 LPG, propane, into isobutene and propene at high yield via direct dehydrogenation, and a method for preparing the same. The present invention provides a dehydrogenation catalyst for converting a paraffin-based hydrocarbon having a carbon number of 3 or 4 into an olefin-based hydrocarbon via direct dehydrogenation, the dehydrogenation catalyst including a metal alloy (ZnO-Al$_2$O$_3$) carrier composed of alumina (Al$_2$O$_3$) and zinc oxide (ZnO), and an active metal and an auxiliary active metal which are carried by the metal alloy carrier.

EP 3 175 918 A1

**Description**

**TECHNICAL FIELD**

The present invention relates to a dehydrogenation catalyst and a method for preparing the same, and more particularly, to a dehydrogenation catalyst used for preparing high yields of isobutene and propene from isobutane and propane and a method for preparing the same.

**BACKGROUND ART**

As petrochemicals have become diversified, techniques related to dehydrogenation catalysts and reactions are receiving significant interest as techniques for preparing olefins from natural gas and liquefied petroleum gas (LPG).

Typically, dehydrogenation methods include direct dehydrogenation and oxidative dehydrogenation. In oxidative dehydrogenation, oxygen is introduced with reactants and removes coke produced during dehydrogenation by oxidizing the coke. Side products produced therein may reduce the selectivity to desired isobutene product. Moreover, in direct dehydrogenation, isobutene and hydrogen are produced as isobutane passes through a catalyst layer. Although direct dehydrogenation advantageously has higher selectivity than oxidative dehydrogenation, the coke produced during the reaction covers the active material of the catalyst and is thus a factor in reducing activity. Therefore, a technique for maintaining high catalyst activity while suppressing the coke production of the catalyst is of importance.

Platinum, which typically acts as the point of activity for dehydrogenation catalysts, is easily deactivated at high temperatures, and the activity thereof is easily reduced by carbon deposition. Thus, research is being carried out on catalysts in which metals composed of different substances are added to enable highly selective activity to be maintained over long periods of time.

Moreover, the preparation of catalysts by adding alkali metals such as lithium, potassium, or sodium to increase the thermal stability of the dehydrogenation reaction or by using zinc or magnesium metal to regulate acidity or basicity have been researched, but there has been no mention of using a particular metal to prepare a carrier in the form of an alloy and impregnating the support with an alkali metal to thereby reaction yield (Catalysis Today, Volume 143, Issues 3-4, 2009, 334-340).

Research is also being carried out for enhancing the dispersion of active material, that is, platinum metal, by adding rare earth metals such as lanthanum, cerium, yttrium, and the like as catalyst activity promoters for regulating the interaction between metal and support (Catalysis Today, Volume 164, Issue 1, 2001, 214-220).

Yuming Zhou et. al. (Ind.Eng.Chem.Res. Volume 50, Issue 8, 2011, 4280-4285) observed that a uniform distribution of catalyst activity points is obtained by impregnating an inert support such as alumina with a K, La, Pt, Sn precursor mixture, and observed differences in the amount of chemisorption according to changes in the lanthanum composition, but did not disclose anything regarding the properties of metal carriers.

It has been observed that the introduction of a diluent gas such as nitrogen, carbon dioxide, or steam with reactants allows heat to be provided for maintaining the reaction temperature for dehydrogenation, which is an endothermic reaction, performs the role of a diluting agent and thus enables equilibrium conversion to be achieved by reducing the partial pressures of hydrocarbons and hydrogen, and suppresses carbon deposition (Journal of Molecular Catalysis (CHINA) 1999-03).

Catalyst pore volume and size are critical factors for determining the mass transfer coefficients of the reactants and reaction products. Large pore size may be advantageous for enabling large supports to maintain high catalyst activity, and using large supports having large pores are advantageous for maintaining catalyst activity by suppressing the accumulation of coke (WO2010/076928).

Meanwhile, Korean Patent Application Laid-open Publication No. 1993-0017850 mentions a lanthanum metal catalyst while disclosing a method for preparing isobutene at high selectivities via oxidative dehydrogenation of isobutane, but relates to oxidative dehydrogenation and does not mention the use of a carrier.

Korean Patent Application Laid-open Publication No. 2011-0099112 discloses a technique using a La-Mn/inert support as a method for oxidative dehydrogenation of paraffin-based low hydrocarbons, but relates to oxidative dehydrogenation, and having a short reaction time, has the limitation of low selectivity.

Korean Patent Application Laid-open Publication No. 2008-0114817 discloses a method for preparing propene from propane, but relates to oxidative dehydrogenation and does not mention yield.

As above, prior art papers and patents relating to dehydrogenation catalysts are mostly about the active component of the catalyst or the type of carrier. In order to selectively prepare desired products while increasing or maintaining the dehydrogenation performance of the catalyst, it is important to appropriately select the type and substance of the carrier and the composition of additives, and to optimize the reaction conditions.

## DISCLOSURE OF THE INVENTION

### TECHNICAL PROBLEM

An object of the present invention is to provide a dehydrogenation catalyst for converting isobutane and propane, specifically the C4 LPG, isobutane, and the C3 LPG, propane, into isobutene and propene at high yields via direct dehydrogenation, and a method for preparing the same.

Another object of the present invention is to provide a dehydrogenation catalyst in which the amount of coke deposited is low even when involved with reactions taking place at high temperatures (of about 500°C) and isobutene and propene may be obtained from isobutane and propane for long periods of time at high yields, and a method for preparing the same.

### TECHNICAL SOLUTION

In order to achieve such objectives, the present invention provides a dehydrogenation catalyst for converting a paraffin-based hydrocarbon having a carbon number of 3 or 4 into an olefin-based hydrocarbon via direct dehydrogenation, the dehydrogenation catalyst including a metal alloy ($ZnO$-$Al_2O_3$) carrier composed of alumina ($Al_2O_3$) and zinc oxide ($ZnO$); and an active metal and an auxiliary active metal which are carried by the metal alloy carrier.

A dehydrogenation catalyst is provided, characterized in that the paraffin-based hydrocarbon is isobutane or propane; and the olefin-based hydrocarbon is isobutene or propene.

A dehydrogenation catalyst is provided, characterized in that the zinc oxide content is 1-25 parts by weight with respect to 100 parts by weight of alumina

A dehydrogenation catalyst is provided, characterized in that the active metal is platinum (Pt); and the auxiliary active metal is lanthanum (La) and tin (Sn).

A dehydrogenation catalyst is provided, characterized in that the platinum content is 0.1-5 parts by weight with respect to 100 parts by weight of alumina; the lanthanum content is 0.1-10 parts by weight with respect to 100 parts by weight of alumina; and the tin content is 0.1-10 parts by weight with respect to 100 parts by weight of alumina.

A dehydrogenation catalyst is provided, characterized in that the paraffin-based hydrocarbon contains water vapor such that the mole ratio between the water vapor and the hydrocarbon is 0.1-5 (water vapor/hydrocarbon).

A dehydrogenation catalyst is provided, characterized in that the conversion of the paraffin-based hydrocarbon is at least 50% and the selectivity to the olefin-based hydrocarbon is at least 90% when measured under the conditions below,

[Measurement conditions]

where the conversion of the paraffin-based hydrocarbon and the selectivity to the olefin-based hydrocarbon are measured after performing a dehydration reaction for 120 hours at 500°C and a weight hourly space velocity (WHSV) of 1 $hr^{-1}$.

A dehydrogenation catalyst is provided, characterized in that the amount of carbon deposition is less than 3 wt% when measured under the conditions below,

[Measurement conditions]

where the measurement is via thermogravimetric analysis (TGA) after 5 days of dehydrogenation at 500°C.

In order to achieve other such objectives, the present invention provides a method for preparing a dehydrogenation catalyst for converting a paraffin-based hydrocarbon having a carbon number of 3 or 4 into an olefin-based hydrocarbon via direct dehydrogenation, the method including (a) an operation for preparing a metal alloy ($ZnO$-$Al_2O_3$) carrier by performing, in order, impregnation of an alumina ($Al_2O_3$) support with zinc oxide ($ZnO$), drying, and firing; (b) an operation for preparing a lanthanum/zinc oxide-alumina (La/$ZnO$-$Al_2O_3$) catalyst by performing, in order, impregnation of the metal alloy with lanthanum (La), drying, and firing; (c) an operation for preparing a platinum-lanthanum/zinc oxide-alumina (Pt-La/$ZnO$-$Al_2O_3$) catalyst by performing, in order, impregnation of the lanthanum/zinc oxide-alumina catalyst with platinum (Pt), drying, and firing; and (d) an operation for preparing a tin-platinum-lanthanum/zinc oxide-alumina (Sn-Pt-La/$ZnO$-$Al_2O_3$) catalyst by performing, in order, impregnation of the platinum-lanthanum/zinc oxide-alumina (Pt-La/$ZnO$-$Al_2O_3$) catalyst with tin (Sn), drying, and firing.

### ADVANTAGEOUS EFFECTS

According to the present invention, a dehydrogenation catalyst for converting isobutane or propane into isobutene or propene via direct dehydrogenation may be provided by using a carrier composed of a metal alloy ($ZnO$-$Al_2O_3$) as the carrier and having the carrier carry an active metal and an auxiliary active metal such that isobutene or propene may be obtained at high conversion and high selectivity while continuously maintaining the initial activity even for long reaction times.

Moreover, a method for preparing a dehydrogenation catalyst capable of maximizing yield may be provided by preparing a metal alloy (ZnO-Al$_2$O$_3$) carrier and then impregnating the carrier with an active metal and an auxiliary active metal in a predetermined order.

## MODE FOR CARRYING OUT THE INVENTION

Hereinafter, the present invention will be described in detail with reference to exemplary embodiments thereof. Terms or expressions used in the specification and claims should not be construed as limited to their dictionary definitions. Rather, such terms and expressions may be appropriately defined by the inventors as necessary to best describe the invention, and are to be interpreted as having a meaning suitable for the technical concept of the present invention. Accordingly, the features of the embodiments described herein are merely exemplary and do not represent the full extent of the technical concept of the invention, and thus it is to be understood that various equivalents and modifications thereof may exist within the scope of the invention.

The present inventors conceived of the present invention after discovering that with respect to a dehydrogenation catalyst for converting a paraffin-based hydrocarbon having a carbon number of 3 or 4 into an olefin-based hydrocarbon via direct dehydrogenation, when a metal alloy (ZnO-Al$_2$O$_3$) carrier is prepared in which the activity of the dehydrogenation catalyst is increased by using zinc oxide to reduce the acidity of alumina and thereby adjust the basicity of the surface, and optimal amounts of predetermined active metals and auxiliary active metals are introduced into the alloy, the performance of the dehydrogenation catalyst is optimized according to the order in which the metal components are introduced.

Thus, the present invention discloses a dehydrogenation catalyst for converting a paraffin-based hydrocarbon having a carbon number of 3 or 4 into an olefin-based hydrocarbon via direct dehydrogenation, wherein the catalyst is characterized in that an active metal and an auxiliary active metal are carried by a metal alloy (ZnO-Al$_2$O$_3$) carrier composed of alumina (Al$_2$O$_3$) and zinc oxide (ZnO). The present invention also discloses as an optimal method for preparing the dehydrogenation catalyst, a dehydrogenation catalyst preparation method characterized by including (a) an operation for preparing a metal alloy (ZnO-Al$_2$O$_3$) carrier by performing, in order, impregnation of an alumina (Al$_2$O$_3$) support with zinc oxide (ZnO), drying, and firing; (b) an operation for preparing a lanthanum/zinc oxide-alumina (La/ZnO-Al$_2$O$_3$) catalyst by performing, in order, impregnation of the metal alloy with lanthanum (La), drying, and firing; (c) an operation for preparing a platinum-lanthanum/zinc oxide-alumina (Pt-La/ZnO-Al$_2$O$_3$) catalyst by performing, in order, impregnation of the lanthanum/zinc oxide-alumina catalyst with platinum (Pt), drying, and firing; and (d) an operation for preparing a tin-platinum-lanthanum/zinc oxide-alumina (Sn-Pt-La/ZnO-Al$_2$O$_3$) catalyst by performing, in order, impregnation of the platinum-lanthanum/zinc oxide-alumina (Pt-La/ZnO-Al$_2$O$_3$) catalyst with tin (Sn), drying, and firing.

In the present invention, isobutane or propane is desirably used as a paraffin-based hydrocarbon used as the raw material for the direct dehydrogenation reaction, and isobutane may be the most desirable among C4 liquefied petroleum gasses and propane may be the most desirable among C3 liquefied petroleum gasses. Using the isobutane or the propane as the raw material, isobutene or propene may be prepared through direct dehydrogenation.

In the present invention, it was observed that by adopting alumina as a carrier for the dehydrogenation catalyst, that is, by using a zinc oxide-alumina metal alloy carrier, in which the basicity of the surface has been adjusted by using zinc oxide to reduce the acidity of the alumina, and selecting a catalyst impregnated with an active metal and an auxiliary active metal in order to increase the activity of the catalyst required for the dehydrogenation reaction, the dehydrogenation catalyst may be used to prepare isobutene and propene while maintaining a high conversion of a paraffin-based hydrocarbon and a high selectivity to an olefin-based hydrocarbon for long periods of time. In commercial petrochemical processes for preparing isobutene and propene, there are hardly any cases in which isobutene and propene are produced at high yields for long periods of time without catalyst regeneration. Although regeneration processes are typically used for removing coking, the dehydrogenation catalyst according to the present invention provides a large effect in suppressing catalyst deactivation caused by coking, which is the biggest cause of activity reduction in dehydrogenation catalysts used in high-temperature dehydrogenation, and allows the desired product to be reliably obtained at high yields for long periods of time.

Alumina may have an alpha ($\alpha$), gamma ($\gamma$), eta ($\eta$), delta ($\delta$), or theta ($\theta$) crystal structure. Such crystal structures change according to the method by which lattice oxygen is charged, and the size and surface area of micropores in alumina change according to the synthesis conditions. In the present invention, the alumina is desirably $\gamma$-alumina having a spinel structure of slightly twisted squares, and appropriately, has a specific surface area (Brunauer, Emmett and Teller (BET)) of 195-215 m$^2$g$^{-1}$.

In order to improve and maintain for long periods of time the conversion of the paraffin-based hydrocarbon and the selectivity to the olefin-based hydrocarbon, the content of the zinc oxide alloyed with the alumina is desirably 1-25 parts by weight, more desirably 5-15 parts by weight, even more desirably 8-12 parts by weight, and most desirably 9-11 parts by weight with respect to 100 parts by weight of the alumina. Adjustment of surface basicity through alumina acidity reduction is most convenient in this zinc oxide content range.

Although both ion-exchange and impregnation methods may be used for preparing the carrier composed of alumina and zinc oxide, it is desirable to use an initial wet impregnation method which is convenient for the adjustment of surface basicity through alumina acidity reduction. For example, preparation may involve impregnating an alumina support with a zinc oxide precursor, zinc nitrate hexahydrate ($Zn(NO_3)_2 \cdot 6H_2O$), drying for 12-36 hours at 60-120°C in a dryer, and then firing in the presence of oxygen at 500-600°C and reducing in the presence of hydrogen for 2-4 hours.

In the present invention, platinum as the active metal and lanthanum and tin as the auxiliary active metals may be carried by the metal alloy carrier.

Lanthanum is carried in order to enhance the thermal stability of the catalyst in the endothermic dehydrogenation reaction and suppress the catalyst deactivation caused by coking. When introduced in an optimum content, the performance of the dehydrogenation catalyst may be optimized. The lanthanum content for optimizing catalyst performance is desirably 0.1-10 parts by weight, more desirably 0.5-5 parts by weight, and most desirably 1-3 parts by weight with respect to 100 parts by weight of the alumina.

The introduction of lanthanum may be performed, for example, by impregnating the metal alloy carrier with a lanthanum precursor, lanthanum nitrate hexahydrate ($La(NO_3)_3 \cdot 6H_2O$), drying for 12-36 hours at 60-120°C in a dryer, and then firing in the presence of oxygen at 500-600°C and reducing in the presence of hydrogen for 2-4 hours.

In the present invention, platinum is carried to act as the activation point of the catalyst, tin is carried-as a cocatalyst for preventing the platinum from being easily deactivated at high temperature-to suppress side reactions to the dehydrogenation reaction, that is, hydrogenolysis, oligomerization, and coke formation of the catalyst surface by performing the role of a catalyst activity promoter and thus reducing the catalyst deactivation rate and increasing catalyst stability.

According to the present invention, when optimum amounts of platinum and tin are introduced into the lanthanum/zinc oxide-alumina ($La/ZnO-Al_2O_3$) catalyst introduced as above in optimum amounts with the carrier-in which alumina and zinc oxide are alloyed-as the base, coke formation is suppressed even in high temperature reactions, and thus desired products may be produced at high yields and deactivation may be suppressed for long periods of time. To achieve this, desirably 0.1-5 parts by weight, more desirably 0.1-2 parts by weight, and most desirably 0.5-1.5 parts by weight of platinum may be introduced with respect to 100 parts by weight of alumina. Moreover, 0.1-10 parts by weight, more desirably 1-5 parts by weight, and most desirably 2-4 parts by weight of tin may be introduced with respect to 100 parts by weight of alumina.

The introduction of platinum may be performed, for example, by impregnating the lanthanum/zinc oxide-alumina catalyst with a platinum precursor, chloroplatinic acid hexahydrate ($H_2PtCl_6 \cdot 6H_2O$), drying for 12-36 hours at 60-120°C in a dryer, and then firing in the presence of oxygen at 500-600°C and reducing in the presence of hydrogen for 2-4 hours to thereby prepare a platinum-lanthanum/zinc oxide-alumina ($Pt-La/ZnO-Al_2O_3$) catalyst. The introduction of tin may be prepared, for example, by impregnating the platinum-lanthanum/zinc oxide-alumina catalyst with a tin precursor, tin-acetylacetonate, and drying for 12-36 hours at 60-120°C in a dryer, and then firing in the presence of oxygen at 500-600°C and reducing in the presence of hydrogen for 2-4 hours to thereby prepare a tin-platinum-lanthanum/zinc oxide-alumina ($Sn-Pt-La/ZnO-Al_2O_3$) catalyst.

Here, as above, the introduction of the active metal and the auxiliary active metals is desirably performed in the following order: lanthanum, platinum, tin. That is, when introduced in an order other than lanthanum, platinum, tin, it was observed that there is not a significant improvement in yield compared to cases in which the metal alloy carrier is not used.

Meanwhile, in the direct dehydrogenation reaction using the dehydrogenation catalyst according to the present invention, it is particularly desirable in terms of yield when the paraffin-based hydrocarbon used as the reactant contains a predetermined amount of moisture (water vapor). By introducing water vapor with the reactant, heat required for maintaining the reaction temperature may be provided, the water vapor may perform the role of a diluent by reducing the partial pressure of the hydrocarbon and hydrogen such that equilibrium conversion is achieved, and deposition of carbon formed during the reaction may be more removed with greater efficiency.

Thus, according to an exemplary embodiment of the present invention, the mole ratio between the hydrocarbon and water vapor contained in the paraffin-based hydrocarbon used in the dehydrogenation reaction may be 0.1-5 (water vapor/hydrocarbon), more desirably 1-3, and most desirably 1.5-2.5. When the water vapor/hydrocarbon mole ratio is less than 0.1, the improvement in yield compared to the case in which hydrocarbon free of water vapor is used may not be significant, and when the mole ratio exceeds 5, it may be difficult to expect further improvements in yield.

As such, the dehydrogenation catalyst prepared by impregnating the metal alloy carrier with optimum amounts of-in order-lanthanum, platinum, and tin enables the side reaction-coking-to be largely suppressed even at high temperatures of about 470-520°C, allows isobutene and propene to be prepared at high yields, and allows isobutene and propene to be obtained at high yields without deactivation for long periods of time. In particular, the yield may be maximized when using the hydrocarbon reactant containing an appropriate amount of water vapor. For example, the dehydrogenation catalyst according to the present invention may enable the paraffin-based hydrocarbon conversion to be at least 50% and the selectivity to the olefin-based hydrocarbon to be at least 90% when measured after performing the dehydrogenation reaction for 120 hours at a weight hourly space velocity (WHSV) of 1 hr$^{-1}$ and a temperature of 500°C. Moreover, the amount of carbon deposition measured via thermogravimetric analysis (TGA) after 5 days of dehydrogenation at 500°C may be less than 3 wt%.

The dehydrogenation catalyst according to the present invention may be used in a reaction for converting a paraffin-based hydrocarbon having a carbon number of 3 or 4 into an olefin-based hydrocarbon via direct dehydrogenation. For example, the olefin-based hydrocarbon may be prepared by using a fixed bed reactor to react the paraffin-based hydrocarbon raw material at a reaction temperature in the range of 450-550°C, desirably 470-520°C, at a weight hourly space velocity (WHSV) condition of 0.5-5 hr$^{-1}$. When the reaction temperature is under 450°C, conversion of the paraffin-based hydrocarbon may be reduced, and when above 550°C, conversion of the paraffin-based hydrocarbon is increased, but the selectivity to the olefin-based hydrocarbon may be significantly reduced due to the formation of side reaction products. The weight hourly space velocity (WHSV) indicates the net mass flow rate of the paraffin-based hydrocarbon in the raw material with respect to the mass of the catalyst involved in the reaction, and may be measured by using the initial mass of the catalyst and adjusting the flow rate of the paraffin-based hydrocarbon. When the weight hourly space velocity is less than 0.5 h$^{-1}$, the conversion may increase but it may be difficult to produce the olefin-based hydrocarbon in large quantities, and when exceeding 5 h$^{-1}$, the conversion is reduced and catalyst deactivation and a reduction in catalyst lifetime may occur.

Hereinafter, the present invention is described in greater detail with reference to specific examples.

### Example 1

$\gamma$-alumina having a surface area of 212.91 m$^2$g$^{-1}$ was prepared as a catalyst carrier support by firing spherical alumina (Al$_2$O$_3$, Sigma Aldrich) in a firing furnace at 550°C for 6 hours. The specific surface area and pore volume of the prepared $\gamma$-alumina are shown in Table 1. Next, using the fired $\gamma$-alumina as a support, 15 of the $\gamma$-alumina was impregnated with an aqueous solution composed of 8.09 g of zinc nitrate hexahydrate dissolved in 5.85 g of distilled water, and then dried in an 80°C oven for 24 hours. A zinc oxide-alumina (ZnO-Al$_2$O$_3$) metal alloy carrier containing 10 parts by weight of zinc oxide to 100 parts by weight of alumina was prepared by firing the dried catalyst in an air furnace at 550°C for 6 hours. Next, 15 g of the prepared metal alloy carrier was impregnated with an aqueous solution composed of 0.94 g of lanthanum nitrate hexahydrate dissolved in 11.49 g of distilled water, and then dried in an 80°C oven for 24 hours. A lanthanum/zinc oxide-alumina (La/ZnO-Al$_2$O$_3$) catalyst containing 2 parts by weight of lanthanum to 100 parts by weight of alumina was prepared by firing the dried catalyst in an air furnace at 550°C for 6 hours. Next, 15 g of the prepared lanthanum/zinc oxide-alumina catalyst was impregnated with an aqueous solution composed of 0.33 g of H$_2$PtCl$_6$·6H$_2$O dissolved in 12.37 g of distilled water, and then dried in an 80°C oven for 24 hours. A platinum-lanthanum/zinc oxide-alumina (Pt-La/ZnO-Al$_2$O$_3$) catalyst containing 1 part by weight of platinum to 100 parts by weight of alumina was prepared by firing the dried catalyst in an air furnace at 550°C for 6 hours. Next, the prepared platinum-lanthanum/zinc oxide-alumina catalyst was impregnated with a solution composed of 1.30 g of tin acetyl acetonate dissolved in 11.27 g of acetone, and then dried in an 80°C oven for 24 hours. A tin-platinum-lanthanum/zinc oxide-alumina catalyst (Sn-Pt-La/ZnO-Al$_2$O$_3$) containing 3 parts by weight of tin to 100 parts by weight of alumina was prepared by firing the dried catalyst in an air furnace at 550°C for 6 hours.

[Table 1]

| Specimen | Specific surface area (m$^2$/g) | Pore volume (cm$^2$/g) |
|---|---|---|
| $\gamma$-alumina | 212.91 | 0.5651 |

### Example 2

Other than excluding the operation in Example 1 of impregnating with lanthanum, the same method as in Example 1 was used to prepare a tin-platinum/zinc oxide-alumina (Sn-Pt/ZnO-Al$_2$O$_3$) catalyst.

### Example 3

Other than excluding the operation in Example 1 in which 5.47 g of zinc nitrate hexahydrate is used so that the zinc oxide content is 7 parts by weight with respect to 100 parts by weight of alumina, the same method as in Example 1 was used to prepare a tin-platinum-lanthanum/zinc oxide-alumina (Sn-Pt-La/ZnO-Al$_2$O$_3$) catalyst.

### Example 4

Other than excluding the operation in Example 1 in which 12.89 g of zinc nitrate hexahydrate is used so that the zinc oxide content is 15 parts by weight with respect to 100 parts by weight of alumina, the same method as in Example 1 was used to prepare a tin-platinum-lanthanum/zinc oxide-alumina (Sn-Pt-La/ZnO-Al$_2$O$_3$) catalyst.

**Comparative Example 1**

Other than excluding the operation in Example 1 of alloying with zinc oxide, the same method as in Example 1 was used to prepare a tin-platinum/alumina (Sn-Pt/Al$_2$O$_3$) catalyst.

**Example 5**

Other than excluding the operation in Example 1 in which 0.11 g of lanthanum nitrate hexahydrate is used so that the lanthanum content is 0.25 parts by weight with respect to 100 parts by weight of alumina, the same method as in Example 1 was used to prepare a tin-platinum-lanthanum/zinc oxide-alumina (Sn-Pt-La/ZnO-Al$_2$O$_3$) catalyst.

**Example 6**

Other than excluding the operation in Example 1 in which 0.23 g of lanthanum nitrate hexahydrate is used so that the lanthanum content is 0.5 parts by weight with respect to 100 parts by weight of alumina, the same method as in Example 1 was used to prepare a tin-platinum-lanthanum/zinc oxide-alumina (Sn-Pt-La/ZnO-Al$_2$O$_3$) catalyst.

**Example 7**

Other than excluding the operation in Example 1 in which 0.47 g of lanthanum nitrate hexahydrate is used so that the lanthanum content is 1 part by weight with respect to 100 parts by weight of alumina, the same method as in Example 1 was used to prepare a tin-platinum-lanthanum/zinc oxide-alumina (Sn-Pt-La/ZnO-Al$_2$O$_3$) catalyst.

**Example 8**

Other than excluding the operation in Example 1 in which 1.44 g of lanthanum nitrate hexahydrate is used so that the lanthanum content is 3 parts by weight with respect to 100 parts by weight of alumina, the same method as in Example 1 was used to prepare a tin-platinum-lanthanum/zinc oxide-alumina (Sn-Pt-La/ZnO-Al$_2$O$_3$) catalyst.

**Example 9**

Other than using yttrium nitrate hexahydrate instead of lanthanum nitrate hexahydrate in Example 1, the same method as in Example 1 was used to prepare a tin-platinum-yttrium/zinc oxide-alumina (Sn-Pt-Yt/ZnO-Al$_2$O$_3$) catalyst.

**Example 10**

Other than changing the order in which the metal alloy carrier is impregnated with the metal components in Example 1 to lanthanum, tin, platinum, the same method as in Example 1 was used to prepare a platinum-tin-lanthanum/zinc oxide-alumina (Pt-Sn-La/ZnO-Al$_2$O$_3$) catalyst.

**Example 11**

Other than changing the order in which the metal alloy carrier is impregnated with the metal components in Example 1 to platinum, lanthanum, tin, the same method as in Example 1 was used to prepare a tin-lanthanum-platinum/zinc oxide-alumina (Sn-La-Pt/ZnO-Al$_2$O$_3$) catalyst.

**Example 12**

Other than changing the order in which the metal alloy carrier is impregnated with the metal components in Example 1 to platinum, tin, lanthanum, the same method as in Example 1 was used to prepare a lanthanum-platinum-tin/zinc oxide-alumina (La-Pt-Sn/ZnO-Al$_2$O$_3$) catalyst.

**Example 13**

Other than changing the order in which the metal alloy carrier is impregnated with the metal components in Example 1 to tin, lanthanum, platinum, the same method as in Example 1 was used to prepare a platinum-tin-lanthanum/zinc oxide-alumina (Pt-Sn-La /ZnO-Al$_2$O$_3$) catalyst.

## Example 14

Other than changing the order in which the metal alloy carrier is impregnated with the metal components in Example 1 to tin, platinum, lanthanum, the same method as in Example 1 was used to prepare a lanthanum-tin-platinum/zinc oxide-alumina (La-Sn-Pt/ZnO-Al$_2$O$_3$) catalyst.

The compositions (unit: parts by weight) of the dehydrogenation catalysts according to the above Examples and Comparative Example are shown in Table 2 below.

[Table 2]

| Example | La | Pt | Sn | ZnO | Order of impregnation |
|---|---|---|---|---|---|
| Example 1 | 2 | 1 | 3 | 10 | La→Pt→Sn |
| Example 2 | - | 1 | 3 | 10 | |
| Example 3 | 2 | 1 | 3 | 7 | |
| Example 4 | 2 | 1 | 3 | 15 | |
| Comparative Example 1 | 2 | 1 | 3 | - | |
| Example 5 | 0.25 | 1 | 3 | 10 | |
| Example 6 | 0.5 | 1 | 3 | 10 | |
| Example 7 | 1 | 1 | 3 | 10 | |
| Example 8 | 3 | 1 | 3 | 10 | |
| Example 9 | 2 (Y) | 1 | 3 | 10 | |
| Example 10 | 2 | 1 | 3 | 10 | La→Sn→Pt |
| Example 11 | 2 | 1 | 3 | 10 | Pt→La→Sn |
| Example 12 | 2 | 1 | 3 | 10 | Pt→Sn→La |
| Example 13 | 2 | 1 | 3 | 10 | Sn→La→Pt |
| Example 14 | 2 | 1 | 3 | 10 | Sn→Pt→La |

## Experimental Example 1

Preparation reactions such as those below were performed to analyze the performance of dehydrogenation catalysts prepared according to the above Examples and Comparative Example.

[Example preparation reaction]

Isobutene was prepared via a dehydrogenation reaction by charging a stainless steel (SUS) reactor with 5 g of a prepared catalyst and supplying nitrogen, isobutane, and water vapor. The reaction was performed by fixing the mole ratio of isobutane to nitrogen at 4:6 and the mole ratio of water vapor to isobutane at 2:1. Dehydrogenation was carried out under normal pressure conditions at a reaction temperature of 500°C and a weight hourly space velocity (WHSV) of 1 hr$^{-1}$. The water vapor was introduced into the reactor in the gas phase after passing through a 130°C preheater, and thus all of the reactants underwent gas-phase reactions. While continuing the dehydrogenation reaction, reaction products were automatically analyzed using gas chromatography, and the results obtained are displayed below in Tables 3 to 5. Here, the isobutane conversion and the selectivity to isobutene were calculated according to the Formulas 1 and 2 below.

[Formula 1]

$$\text{Isobutane conversion (\%)} = \frac{\text{Moles of isobutane converted into product}}{\text{Moles of isobutane supplied}} \times 100$$

[Formula 2]

$$\text{Selectivity to isobutene (\%)} = \frac{\text{Moles converted into isobutene}}{\text{Moles of isobutane converted into product}} \times 100$$

[Table 3]

| Example | Reaction time: 1 hour | |
| --- | --- | --- |
| | Conversion (%) | Selectivity (%) |
| Example 1 | 52.4 | 90.0 |
| Example 2 | 45.5 | 83.0 |
| Example 3 | 45.8 | 84.4 |
| Example 4 | 44.1 | 83.9 |
| Comparative Example 1 | 32.5 | 79.6 |

[Table 4]

| Example | Reaction time: 1 hour | | Reaction time: 24 hours | |
| --- | --- | --- | --- | --- |
| | Conversion (%) | Selectivity (%) | Conversion (%) | Selectivity (%) |
| Example 1 | 52.4 | 90.0 | 56.5 | 93.5 |
| Example 5 | 50.9 | 81.0 | 42.8 | 83.3 |
| Example 6 | 50.2 | 84.1 | 29.4 | 90.2 |
| Example 7 | 48.6 | 90.1 | 33.0 | 88.7 |
| Example 8 | 49.8 | 91.6 | 35.8 | 92.1 |
| Example 9 | 46.1 | 92.8 | 44.3 | 96.9 |

[Table 5]

| Example | Reaction time: 1 hour | |
| --- | --- | --- |
| | Conversion (%) | Selectivity (%) |
| Example 1 | 52.4 | 90.0 |
| Example 10 | 44.2 | 82.4 |
| Example 11 | 43.7 | 84.3 |
| Example 12 | 45.3 | 82.6 |
| Example 13 | 42.9 | 81.5 |
| Example 14 | 40.2 | 79.8 |

First, referring to Table 3, the catalysts (Examples 1 to 4) prepared using the zinc oxide-alumina metal alloy carrier according to the present invention exhibited high initial activity at 500°C conditions, but the catalyst (Comparative Example 1) which did not use the metal alloy carrier was observed to have significantly reduced conversion and selectivity. Moreover, when the catalyst prepared by further carrying lanthanum metal was used, large improvements to conversion and selectivity were observed (compare Examples 1 and 2). Furthermore, it may be seen that catalyst performance is maximized when the zinc oxide content is about 10 parts by weight with respect to 100 parts by weight of alumina (compare Examples 1, 3, and 4).

Referring to Table 4, although carrying lanthanum metal improved the initial conversion and selectivity, after the experiment was carried out for a long period of time of about 24 hours, there were observed differences in activity reduction according to lanthanum metal content. When the lanthanum content was on the level of about 2 parts by weight with respect to 100 parts by weight of alumina (Example 1), a conversion of at least 50% and a selectivity of at least 90% were maintained, and thus it was observed that the activity was maintained at a high level for a long period of time. Meanwhile, it was observed that a conversion of at least 40% and a selectivity of at least 90% were also maintained in the case of the catalyst carrying yttrium instead of lanthanum (Example 9).

Referring to Table 5, it may be seen that there are differences in initial conversion and selectivity depending on the order in which the carried metal components are carried. That is, compared to when lanthanum, platinum, and tin are carried in this order by the metal alloy carrier according to the present invention (Example 1), significant reductions in the initial conversion and selectivity were observed when the order was changed (Examples 10 to 14).

**Experimental Example 2**

In order to confirm whether further carrying lanthanum allows the dehydrogenation catalyst according to the present invention to be used in the preparation of olefin at high yields without long periods of deactivation, preparation reactions such as above were performed for the dehydrogenation catalysts prepared according to Examples 1 to 9, and long lifetime tests were performed for the catalysts by measuring the conversion and selectivity for predetermined time periods up to 120 hours from the start of the dehydrogenation reaction. The results thereof are displayed in Table 6 below. In addition, carbon deposition amounts for the catalysts were measured via thermogravimetric analysis (TGA) and displayed in Table 7.

[Table 6]

| Example | Yield | Reaction time (hr) | | | | | |
|---|---|---|---|---|---|---|---|
| | | 1 | 24 | 48 | 72 | 96 | 120 |
| Example 1 | Conversion (%) | 52.4 | 56.5 | 54.4 | 54.8 | 47.8 | 54.3 |
| | Selectivity (%) | 90.0 | 93.5 | 94.4 | 94.8 | 95.8 | 93.7 |
| Example 9 | Conversion (%) | 46.1 | 44.3 | 51.7 | 43.2 | 33.7 | 26.7 |
| | Selectivity (%) | 92.8 | 96.9 | 93.8 | 95.1 | 95.2 | 92.8 |

[Table 7]

| Example | Carbon deposition amount (wt%) |
|---|---|
| Example 1 | 2.41 |
| Example 9 | 5.38 |

Referring to Table 6, by carrying out the dehydrogenation catalyst long lifetime test using the catalyst prepared by further adopting lanthanum in the metal alloy carrier according to the present invention, it was observed that at 500°C, the same level of yield was maintained, that is, the activity was maintained for long periods of time. Specifically, after 120 hours of the dehydrogenation long lifetime test, large differences in catalyst performance were observed. In the case of the catalyst further adopting yttrium rather than lanthanum (Example 9), the conversion started to drop significantly after 96 hours of dehydrogenation, whereas in the case of the catalyst further adopting lanthanum (Example 1), it was observed that a conversion of at least 50% and a selectivity of at least 90% could be continuously maintained when carrying out dehydrogenation under conditions in which the isobutane weight hourly space velocity was 1 hr$^{-1}$.

Moreover, referring to Table 7, the amount of carbon deposition was extremely low in the case of the catalyst in which lanthanum was further adopted (Example 1), whereas in the case of the catalyst in which an equivalent content of yttrium was further adopted instead of lanthanum (Example 9), the amount of carbon deposition which occurred was observed to be at least two times that of the catalyst in which lanthanum was further adopted. This indicates that when isobutene is prepared at high yields, coking, which is a side reaction, may be largely suppressed.

## Experimental Example 3

With respect to the dehydrogenation catalyst according to the present invention, in order to confirm whether the dehydrogenation performance is maintained when propane is used as the reactant instead of isobutane, a dehydrogenation reaction was performed for the dehydrogenation catalyst prepared according to Example 1 using the same conditions as the above preparation method, except that propane was used as the reactant instead of isobutane. A long lifetime test of the catalyst was performed by measuring the conversion and selectivity for predetermined time periods up to 24 hours from the start of the dehydrogenation reaction, and the results thereof are displayed in Table 8 below.

[Table 8]

| Example | Yield | Reaction time (hr) | | | | | | | | |
|---|---|---|---|---|---|---|---|---|---|---|
| | | 1 | 3 | 6 | 9 | 12 | 15 | 18 | 21 | 24 |
| Example 1 | Conversion (%) | 49.2 | 48.8 | 47.8 | 46.4 | 45.6 | 45.9 | 45.7 | 45.1 | 45.2 |
| | Selectivity (%) | 91.6 | 91.9 | 93.5 | 95.0 | 94.7 | 90.3 | 94.1 | 91.7 | 91.4 |

Referring to Table 8, after performing the dehydrogenation reaction and the 24 hour lifetime test using the catalyst prepared according to the present invention and using propane as the reactant, it was observed that a conversion of at least 45% and a selectivity of at least 90% could be maintained.

## Example 4

With respect to the dehydrogenation catalyst according to the present invention, in order to observe the difference in the dehydrogenation performance according to the water vapor content in the hydrocarbon reactant, dehydrogenation reactions were performed for the dehydrogenation catalyst prepared according to Example 1 using the same conditions as the above preparation method, except that the water vapor/isobutane mole ratios were further adjusted to be 0, 0.2, 1, 3, and 5, respectively. The conversions and yields were measured after 1 hour of dehydrogenation, and the results are displayed in Table 9 below. For comparison, the result for when the water vapor/isobutane mole ratio is 2 (see Examples 1 and 2) are also displayed.

[Table 9]

| Water vapor/hydrocarbon mole ratio | Conversion (%) | Selectivity (%) |
|---|---|---|
| 0 | 43.3 | 80.1 |
| 0.2 | 45.4 | 82.5 |
| 1 | 49.2 | 85.3 |
| 2 | 52.4 | 90.0 |
| 3 | 52.1 | 88.4 |
| 5 | 51.6 | 89.3 |

Referring to Table 9, when the test was carried out with a water vapor to hydrocarbon mole ratio of at most 5, the conversion and selectivity improved as the water vapor/hydrocarbon mole ratio increased, and it may be seen that a mole ratio of about 2 at which equilibrium is reached is most desirable.

Although the exemplary embodiments of the present invention have been described in order to achieve the technical objectives, it is understood that various changes and modifications can be made by one with ordinary skilled in the art within the spirit and scope of the present invention as hereinafter claimed.

## Claims

1. A dehydrogenation catalyst for converting a paraffin-based hydrocarbon having a carbon number of 3 or 4 into an olefin-based hydrocarbon via direct dehydrogenation, the dehydrogenation catalyst comprising:

a metal alloy (ZnO-Al$_2$O$_3$) carrier composed of alumina (Al$_2$O$_3$) and zinc oxide (ZnO); and
an active metal and an auxiliary active metal which are carried by the metal alloy carrier.

2. The dehydrogenation catalyst of claim 1, wherein:

the paraffin-based hydrocarbon is isobutane or propane; and
the olefin-based hydrocarbon is isobutene or propene.

3. The dehydrogenation catalyst of claim 1, wherein the zinc oxide content is 1-25 parts by weight with respect to 100 parts by weight of alumina.

4. The dehydrogenation catalyst of claim 1, wherein:

the active metal is platinum (Pt); and
the auxiliary active metal is lanthanum (La) and tin (Sn).

5. The dehydrogenation catalyst of claim 4, wherein:

the platinum content is 0.1-5 parts by weight with respect to 100 parts by weight of alumina;
the lanthanum content is 0.1-10 parts by weight with respect to 100 parts by weight of alumina; and
the tin content is 0.1-10 parts by weight with respect to 100 parts by weight of alumina.

6. The dehydrogenation catalyst of claim 1, wherein the paraffin-based hydrocarbon contains water vapor such that the mole ratio between the water vapor and the hydrocarbon is 0.1-5 (water vapor/hydrocarbon).

7. The dehydrogenation catalyst of claim 6, wherein the conversion of the paraffin-based hydrocarbon is at least 50% and the selectivity to the olefin-based hydrocarbon is at least 90% when measured under the conditions below,
[Measurement conditions]
where the conversion of the paraffin-based hydrocarbon and the selectivity to the olefin-based hydrocarbon are measured after performing a dehydration reaction for 120 hours at 500°C and a weight hourly space velocity (WHSV) of 1 hr$^{-1}$.

8. The dehydrogenation catalyst of claim 1, wherein the amount of carbon deposition is less than 3 wt% when measured under the conditions below,
[Measurement conditions]
where the measurement is via thermogravimetric analysis (TGA) after 5 days of dehydrogenation at 500°C.

9. A method for preparing a dehydrogenation catalyst for converting a paraffin-based hydrocarbon having a carbon number of 3 or 4 into an olefin-based hydrocarbon via direct dehydrogenation, the method comprising:

(a) an operation for preparing a metal alloy (ZnO-Al$_2$O$_3$) carrier by performing, in order, impregnation of an alumina (Al$_2$O$_3$) support with zinc oxide (ZnO), drying, and firing;
(b) an operation for preparing a lanthanum/zinc oxide-alumina (La/ZnO-Al$_2$O$_3$) catalyst by performing, in order, impregnation of the metal alloy with lanthanum (La), drying, and firing;
(c) an operation for preparing a platinum-lanthanum/zinc oxide-alumina (Pt-La/ZnO-Al$_2$O$_3$) catalyst by performing, in order, impregnation of the lanthanum/zinc oxide-alumina catalyst with platinum (Pt), drying, and firing; and
(d) an operation for preparing a tin-platinum-lanthanum/zinc oxide-alumina (Sn-Pt-La/ZnO-Al$_2$O$_3$) catalyst by performing, in order, impregnation of the platinum-lanthanum/zinc oxide-alumina (Pt-La/ZnO-Al$_2$O$_3$) catalyst with tin (Sn), drying, and firing.

## INTERNATIONAL SEARCH REPORT

| International application No. |
|---|
| **PCT/KR2015/007671** |

**A. CLASSIFICATION OF SUBJECT MATTER**

*B01J 23/62(2006.01)i, B01J 23/63(2006.01)i, C07C 4/06(2006.01)i, B01J 37/02(2006.01)i*

According to International Patent Classification (IPC) or to both national classification and IPC

**B. FIELDS SEARCHED**

Minimum documentation searched (classification system followed by classification symbols)

B01J 23/62; C07C 11/02; B01J 23/80; B01J 35/04; B01J 21/04; C07B 61/00; B01J 37/02; B01J 23/755; B01J 23/60; B01J 23/63; C07C 4/06

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)

eKOMPASS (KIPO internal) & Keywords: catalyst, catalyst, alumina, alumina, Al2O3, zinc oxide, zinc oxide, ZnO

**C. DOCUMENTS CONSIDERED TO BE RELEVANT**

| Category* | Citation of document, with indication, where appropriate, of the relevant passages | Relevant to claim No. |
|---|---|---|
| X | KR 10-2014-0068606 A (LOTTE CHEMICAL CORPORATION) 09 June 2014<br>See abstract; paragraphs [0039] to [0044]; claims 1-10; and the examples. | 1-9 |
| A | KR 10-2004-0027888 A (JOHNSON MATTHEY PUBLIC LIMITED COMPANY)<br>01 April 2004<br>See abstract; and claims 1-7, 10-15. | 1-9 |
| A | JP 07-206718 A (PHILLIPS PETROLEUM COMPANY) 08 August 1995<br>See abstract; and claims 1-13. | 1-9 |
| A | US 4088607 A (WEIDENBACH, Guenther et al.) 09 May 1978<br>See abstract; and claims 1-17. | 1-9 |
| A | JP 2011-529781 A (UHDE GMBH.) 15 December 2011<br>See abstract; and claims 1-12. | 1-9 |

| ☐ Further documents are listed in the continuation of Box C. | ☒ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11 SEPTEMBER 2015 (11.09.2015) | **11 SEPTEMBER 2015 (11.09.2015)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea<br>Facsimile No. 82-42-472-7140 | Telephone No. |

Form PCT/ISA/210 (second sheet) (July 2009)

13

# EP 3 175 918 A1

| Patent document cited in search report | Publication date | Patent family member | Publication date |
|---|---|---|---|
| KR 10-2014-0068606 A | 09/06/2014 | KR 10-1445241 B1 | 29/09/2014 |
| KR 10-2004-0027888 A | 01/04/2004 | AU 2002-317336 A8 | 24/02/2003 |
| | | CA 2456530 A1 | 20/02/2003 |
| | | EP 1414570 A2 | 06/05/2004 |
| | | EP 1414570 B1 | 03/09/2014 |
| | | JP 2004-537407 A | 16/12/2004 |
| | | JP 4391230 B2 | 24/12/2009 |
| | | TW 229661 A | 21/03/2005 |
| | | TW 229661 B | 21/03/2005 |
| | | US 2004-0266612 A1 | 30/12/2004 |
| | | US 7375049 B2 | 20/05/2008 |
| | | WO 03-013728 A2 | 20/02/2003 |
| | | WO 03-013728 A3 | 24/04/2003 |
| JP 07-206718 A | 08/08/1995 | AT 148085 T | 15/02/1997 |
| | | AU 2849592 A | 02/09/1993 |
| | | AU 649173 B2 | 12/05/1994 |
| | | BR 9205158 A | 31/08/1993 |
| | | CA 2081797 A1 | 27/08/1993 |
| | | CA 2081797 C | 07/07/1998 |
| | | DE 69307554 D1 | 06/03/1997 |
| | | DE 69307554 T2 | 15/05/1997 |
| | | DK 0557982 T3 | 10/02/1997 |
| | | EP 0557982 A2 | 01/09/1993 |
| | | EP 0557982 A3 | 09/11/1994 |
| | | EP 0557982 B1 | 22/01/1997 |
| | | ES 2097938 T3 | 16/04/1997 |
| | | FI 103786 B1 | 30/09/1999 |
| | | FI 930861 A0 | 25/02/1993 |
| | | FI 930861 D0 | 25/02/1993 |
| | | JP 2815275 B2 | 27/10/1998 |
| | | MX 9207111 A1 | 01/09/1993 |
| | | NO 305361 B1 | 18/05/1999 |
| | | NO 930685 A | 27/08/1993 |
| | | NO 930685 D0 | 25/02/1993 |
| | | US 5220091 A | 15/06/1993 |
| US 4088607 A | 09/05/1978 | US 4005177 A | 25/01/1977 |
| JP 2011-529781 A | 15/12/2011 | AR 073188 A1 | 20/10/2010 |
| | | CA 2733278 A1 | 11/02/2010 |
| | | CN 102112224 A | 29/06/2011 |
| | | DE 102008036724 A1 | 11/02/2010 |
| | | EP 2331256 A1 | 15/06/2011 |
| | | KR 10-2011-0038178 A | 13/04/2011 |
| | | MX 2011001403 A | 30/05/2011 |
| | | RU 2011105458 A | 20/09/2012 |
| | | RU 2486007 C2 | 27/06/2013 |

Form PCT/ISA/210 (patent family annex) (July 2009)

**INTERNATIONAL SEARCH REPORT**
Information on patent family members

International application No.

**PCT/KR2015/007671**

| Patent document cited in search report | Publication date | Patent family member | Publication date |
|---|---|---|---|
| | | US 2011-0144400 A1 | 16/06/2011 |
| | | WO 2010-015341 A1 | 11/02/2010 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010076928 A
- KR 19930017850
- KR 20110099112
- KR 20080114817

**Non-patent literature cited in the description**

- *Catalysis Today,* 2009, vol. 143 (3-4), 334-340
- *Catalysis Today,* 2001, vol. 164 (1), 214-220
- **YUMING ZHOU.** *Ind.Eng.Chem.Res.,* 2011, vol. 50 (8), 4280-4285
- *Journal of Molecular Catalysis,* March 1999